# EUROPEAN PATENT APPLICATION

(11) **EP 3 815 676 A1**
(43) Date of publication of application: **05.05.2021**
(21) Application number: 19825699.2
(22) Date of filing: 26.06.2019
(51) Int. Cl.: A61K 9/00, A61K 9/48, A61K 47/02

(54) **SUBCUTANEOUSLY INSERTABLE BIOABSORBABLE CAPSULE FOR DELIVERING DRUG**

(30) Priority: 26.06.2018 KR 20180073124
(71) Applicant: LABnPEOPLE CO.,LTD., Yangju-Si, Gyeonggi-do 11485 (KR)
(72) Inventor: CHO, Sung Youn, Uijeongbu-Si Gyeonggi-do 11812 (KR)
(74) Representative: Jacobi, Markus Alexander
(86) International application number: PCT/KR2019/007737
(87) International publication number: WO 2020/004939

(57) **Abstract**

The subcutaneously implantable bioabsorbable capsule for delivering a drug, according to the present invention, forms an accommodation space for accommodating an active ingredient, and comprises a casing for blocking the accommodation space from the outside so that the active ingredient does not leak to the outside, the casing being made of a bioabsorbable material which can be absorbed into the biological tissue after resolving within the biological tissue to enable the active ingredient in the accommodation space to be discharged to the outside and, thereby, allowing the active ingredient to act inside the body at a regular interval and frequency with one injection without the inconvenience of multiple additional inoculations or the like.

## Description

### [Field of Invention]

The present invention relates to a drug delivery capsule, which is a new type of drug delivery system to be inserted directly into the subcutaneous skin or muscle in a capsule form in order to deliver an active ingredient such as a drug to biological tissue. More particularly, the present invention relates to a subcutaneously implantable and bioabsorbable drug delivery capsule, which includes an accommodation space for an active ingredient such as a drug formed in the capsule and an outer casing made of a bioabsorbable material, thus having a function of starting delivery of the drug after a predetermined time.

### [Background of Invention]

In general, the word vaccine is derived from the Latin word vacca, which means "cow", adopted by Jenner who discovered vaccination. Immunity obtainable by successful inoculation with a vaccine generally includes both humoral immunity (antibody-mediated immunity) and cellular immunity (cell-mediated immunity).

In other words, the goal of vaccination is to induce both humoral immunity, which is an ability to respond immediately when a known vaccination is conducted to generate an antibody and then invasion of the corresponding pathogen may occur in future, as well as the cellular immunity which remembers the corresponding pathogen as an antigen and triggers action of T lymphocytes with functions of phagocytosis and release of cytokine as an inflammation-inducing material upon invasion by a pathogen so as to directly attack the pathogen.

Immunity obtained by an antibody from the mother immediately after birth or resulting from administration of an anti-serum containing an antibody exhibits temporary effects as passive immunity, whereas vaccine-induced immunity is active immunity allowing the immune system to remember a specific pathogen.

Such vaccine is not sufficient for single inoculation because only one inoculation has little effect of antibody formation, instead, complete immunization may be achieved when two, three or more inoculations are executed. The same description is also applied to whole-cell vaccine prepared using the entire pathogen or acellular or subunit vaccine prepared using only a fraction of the pathogen.

Many vaccines are whole-cell vaccines and may also be classified as non-pathogenic vaccines (dead vaccine) which are inactivated not to express pathogenicity, and attenuated vaccines (live vaccine). Dead vaccine is stable because viability of pathogenic microorganisms was removed by chemicals or radiation and has a merit of not exhibiting pathogenicity again in the human body, but requires several additional inoculations due to weak efficacy.

On the other hand, the live vaccine has toxicity attenuated by culturing pathogenic microorganisms in extreme environments or by culturing the same with continuously changing hosts and, if administered to a person with reduced immune function, may recover pathogenicity and cause infectious diseases. However, the live vaccine has an advantage that antibody formation can be completed only by a fewer number of inoculations than the dead vaccine.

In general, except for exceptional cases such as infant oral formulations such as oral polio vaccines, most vaccines have typically been prescribed in the form of intramuscular injection. However, this injection format is not easy for persons to be vaccinated and entails inconvenience of taking several doses every few months or years.

In addition to the inconvenience for the persons to be vaccinated, large-scale livestock farms with tens or hundreds of livestock sometimes raise a serious problem because of livestock epidemics. Therefore, although it is recommended or enforced to give several livestock shots at appropriate times depending on age in weeks or months of the livestock, this is very cumbersome and difficult.

For instance, foot-and-mouth disease caused by infection of cows, pigs, goats and deer, which has been a problem for a long time, can be prevented with more than two doses of the foot-and-mouth vaccine. However, it is not only difficult to manage the inoculation for not every single dose but multiple doses of each individual at exact intervals, but also there is a risk of accident during inoculation of a large number of higher weeks or years-old aged and strong livestock, several weeks or months after first vaccination.

Therefore, there is a need for a technique that does not require additional inoculation by allowing sufficient antibodies to be formed with the first inoculation only to livestock of low weekly or monthly ages, but an appropriate solution has yet to be proposed.

As one of such solutions, the present inventors have studied capsules for delivering active ingredients using a bioabsorbable material so that active ingredients such as vaccines can be desirably activated in biological tissues at regular intervals from the initial inoculation time according to the present invention.

As a technology related to the present invention in terms of configuration, Korean Patent Application No. 10-2014-0053907 disclosed "Drug Delivery Micro Bullets", describing a technique of delivering a drug into the skin by a patch type micro-needle to be attached to the skin and then inserted into the skin, which in turn remains a needle portion (called a bullet) on the skin and allows degradation of the bullet. However, this document merely described conceptual effects of controlling a release rate and amount of the drug depending upon biodegradation rate or the like. Further, "a bullet in a conical structure" was only proposed as a specific means for the above configuration, however, any concrete or specific configuration relevant to "the technique of activating an active ingredient in body tissues after a predetermined time of several weeks to months" to accomplish the object of the present invention as well as effective insertion skills or drug delivery skills was neither proposed nor suggested in the above patent document.

### [Summary of Invention]

### [Technical Problem to be Solved]

Therefore, in order to improve the conventional drug delivery method requiring repeated inoculation or the like, an object of the present invention is to provide a subcutaneously implantable and bioabsorbable drug delivery capsule that may regulate a degradation rate of a casing wall in a specific area to communicate an accommodation space with the outside so as to allow the drug to be delivered to the body only after a predetermined period of time from initial insertion, and finally to allow all ingredients including the casing to be absorbed into the body, thereby eliminating the need for separate removal.

Another object of the present invention is to provide a subcutaneously implantable and bioabsorbable drug delivery capsule that allows each drug to act *in vivo* at regular cycles and times by only single insertion, wherein the drug such as a vaccine or contraceptive pill *in vivo* should be applied repeatedly in the exact period required for specific component to be injected.

A still further object of the present invention is to provide a subcutaneously implantable and bioabsorbable drug delivery capsule, along with a means which can be accurately inserted into a required site such as the inside of the skin or muscle.

### [Technical Solution]

In order to achieve the above objects of the present invention, the subcutaneously implantable and bioabsorbable drug delivery capsule according to the present invention is inserted into subcutaneous biological tissues to deliver an active ingredient to the same, and may include: an accommodation space to receive the active ingredient; and a casing for blocking the accommodation space from the outside so that the active ingredient does not leak to the outside, wherein the casing is made of a bioabsorbable material to be degraded in the biological tissues, thus allowing the active ingredient in the accommodation space to flow to the outside, and then, to be absorbed into the biological tissues.

In this case, the bioabsorbable material constituting the casing of the subcutaneously implantable and bioabsorbable drug delivery capsule (hereinafter, abbrev. to "bioabsorbable drug delivery capsule") may include a biodegradable metal represented by the following Formula 1:

[Formula 1] MgₐZn_{b}X_{c}

wherein each of a, b, and c is a weight percent of each component; a + b + c = 100 wt%; a or b is the largest value in the range of 0 ≤ a ≤ 100, 0 ≤ b ≤ 100, and 0 ≤ c ≤ 40; and X is one or more selected from the group consisting of Ca, Fe, Mn, Si, Na, Zr, Ce and P.

At this time, each of a, b and c in Formula1 is a weight percent of each component, a + b + c = 100% by weight, i) 90≤a≤100, 0≤b≤10, 0≤c≤40 or ii) 0≤ a ≤ 10, 90 ≤ b ≤ 100, 0 ≤ c ≤ 40, and X is at least one selected from the group consisting of Ca, Fe, Mn, Si, Na, Zr, Ce and P.

In this case, the biodegradable metal may be pure Mg including inevitable impurities.

In this case, the biodegradable metal may be pure Zn including inevitable impurities.

On the other hand, at least a portion of the casing is a metal region made of the biodegradable metal, a first leakage point in which the active ingredient in the accommodation space leaks first to the outside may be formed during degradation of the bioabsorbable material in the metal region, while an n^{th} leakage point (n being an integer of 2 or more) in which leakage of the active ingredient sequentially occurs after leakage at the first leakage point may be formed at another site of the casing including the metal region.

In this case, the first leakage point or the n^{th} leakage point may be formed by appropriately arranging a constitutional composition of the biodegradable metal in the metal region, wherein the above constitutional composition allows two or more metal phases to generate a galvanic circuit according to Formula 1 thereby accelerating a degradation rate.

Further, with regard to the first leakage point or the n^{th} leakage point, a thickness of a casing wall between the accommodation space of the casing and the outside may be adjusted to define the thinnest portion (that is, the casing wall portion having the smallest thickness) as the first leakage point, followed by sequentially forming the n^{th} leakage point according to the thickness of the casing wall.

In this case, the accommodation space including the first leakage point or the n^{th} leakage point may include isolated spaces, which are formed in the casing in correspondence with the first leakage point or the n^{th} leakage point, respectively, wherein the active ingredient is isolated from the isolated spaces.

The bioabsorbable drug delivery capsule of the present invention may consist of: a front end made of the biodegradable metal; an expandable part (referred to as "expansion") made of a soft biodegradable polymer material; and at least one nodule made of the biodegradable metal, which is connected to at least one end of the expansion, wherein the expansion is formed in a tube or bag shape that can accommodate the active ingredient therein, and may have a configuration such that the front end or the nodule communicates with both ends of the expansion.

At this time, the front end is provided with a tip member to penetrate the skin while being inserted subcutaneously, and the nodule may have an inlet through which the active ingredient can be injected, as well as a backflow preventing member to prevent the injected active ingredient from being discharged to the outside.

In this case, the inlet of the nodule may have a tubular shape through which an injection needle can penetrate so that the inlet of the nodule may be used by mounting the injection needle of a drug injection means such as a syringe thereon. Further, the backflow preventing means may have a backflow preventing valve structure such as an elastic membrane so that the active ingredient injected from the drug injection means after removing the injection needle can be prevented from being discharged to the outside. In addition, an injection needle seating member may be provided inside a connection part between the front end and the expansion, wherein the injection needle seating member stably secures a skin insertion direction of the front end when mounted on the injection needle.

At this time, the expansion may be made of at least one biodegradable polymer material selected from the group consisting of polydioxanone (PDO), polylactic acid (PLA), poly-L-lactic acid (PLLA), polyglycolic acid (PGA), glycolide-lactide copolymer (PGLA) and glycolide-caprolactone copolymer (PGCL).

### [Effect of Invention]

According to the configuration as described above, the bioabsorbable drug delivery capsule of the present invention may have the following effects.
First, a casing is manufactured using a material which is degraded and absorbed in a living body, and an active ingredient such as a vaccine is injected into an accommodation space inside the casing before insertion into the living body. Simultaneously, it is possible to set a time until a casing wall is degraded and the vaccine or the like in the accommodation space discharges and flows into tissues of the living body ("biological tissues") under a theoretical ground in regard to control of degradation rates of biodegradable metals and experimental results thereof, so that a release amount of drug over time may be regulated by one single insertion without inconvenience of several additional inoculations, or the active ingredient may act in vivo at regular cycles and times.
Second, the entire casing of the capsule is made of a bioabsorbable material and may be degraded and absorbed within the biological tissues such as subcutaneous muscle, thereby avoiding pain and troubles in removing the capsule after use, unlike the intra-uterine contraceptive device used in the art.
Third, unlike typical products used for oral use, it is important to secure hardness of the capsule sufficient to penetrate the skin for in vivo insertion. A biodegradable metal capable of forming a high hardness tip member is applied as a material of the casing, thereby easily inserting the capsule into desired portions in the body.
Fourth, in accordance with the structural features of the capsule to be provided, any appropriate auxiliary means for insertion may also be provided to further facilitate in vivo insertion.

### [Brief Description of Drawings]

FIGS. 1 to 3 illustrate state diagrams in relation to contents of magnesium, zinc and calcium in the biodegradable metal applicable to the subcutaneously implantable and bioabsorbable drug delivery capsule ("bioabsorbable drug delivery capsule") according to the present invention.
FIG. 4 illustrates a graph obtained by measuring the degradation rate of the metal sheet manufactured according to one embodiment of the present invention.
FIG. 5 is a sample photograph of the bioabsorbable drug delivery capsules according to a first embodiment among the preferred embodiments of the present invention.
FIG. 6 is a view of the bioabsorbable drug delivery capsule according to a second embodiment among the preferred embodiments of the present invention.
FIG. 7 is a view of the bioabsorbable drug delivery capsule according to a third embodiment among the preferred embodiments of the present invention.
FIG. 8 illustrates a process of applying the drug delivery capsule in vivo according to the embodiment shown in FIG. 7.
FIG. 9 is a view of the bioabsorbable drug delivery capsule according to a fourth embodiment as a modification of the embodiment shown in FIG. 7.
FIGS. 10 and 11 are graphs illustrating experimental results in relation to effects of antibody formation by comparison between the conventional injection method and the insertion of the capsule according to the first embodiment of the present invention when porcine foot and mouth vaccine was injected.

### [Description of reference numerals]

1:Casing 2: Active ingredient
5: Casing bulkhead 7:Plug
10: Front end 11: Injection needle seat
20: Nodule 21: Inlet
22: Accommodation space 25: Backflow preventing means
30: Expansion 100: Syringe
110: Injection needle

### [Best Mode]

Hereinafter, the configuration of the subcutaneously implantable and bioabsorbable capsule for drug delivery ("bioabsorbable drug delivery capsule") according to the present invention described above will be described in more detail with reference to the accompanying drawings.

On the other hand, in the above and the following description, the term "active ingredient" will be understood to encompass various drugs that require repeated administration from the prior art, and may include, for example, vaccines and, specifically, foot-and-mouth vaccines, but is not limited thereto. In addition to the vaccine, all drugs requiring two or more doses repeatedly such as a contraceptive pill may be included.

The most fundamental feature of the present invention is that the biodegradable metal, which is strong enough to be commercialized as a bone fixation screw or other body implant material, may be easily inserted into the subcutaneous biological tissues. Further, the present invention relates to a technique enabling leakage of an active ingredient in the accommodation space of the capsule to the outside after a desired time, through application of the technology for control of biodegradation rate of the biodegradable metal discovered from studies by the present inventors over the course of many years.

In other words, as disclosed several times in Korean Patent Application No. 10-2017-0044692 entitled "Micro-needle using biodegradable metal" or Korean Patent Application No. 10-2017-0045449 entitled "Biodegradable metal implant", etc., the present inventors have been continuously studying the control of degradation rate of the biodegradable metal comprising magnesium or zinc as a main component or improvement of other physical properties, and some already-disclosed contents are applied to the present invention.

Accordingly, the bioabsorbable drug delivery capsule of the present invention may be manufactured using a biodegradable metal, wherein at least a portion of a casing for preventing leakage of an active ingredient between an accommodation space and the outside is degraded in biological tissues, and the biodegradable metal includes at least one metal selected from Mg and Zn, which are decomposed while generating hydrogen gas according to the following formulae, as a main component:

Mg + 2H2O → Mg(OH)2 + H2 (gas)

Zn + 2H2O → Zn(OH)2 + H2 (gas)

That is, according to the present invention, a metal region of the bioabsorbable drug delivery capsule may be made of magnesium (Mg), zinc (Zn), etc. as a single material, but may also contain a trace of inevitable impurities.

As described above, the bioabsorbable drug delivery capsule according to the present invention may be made of a biodegradable metal as a single material, and may also be manufactured as a combined structure of a part made of a biodegradable metal and another part made of a biodegradable polymer material.

When the capsule is provided in the form of a structure combined with a biodegradable polymer material part, a front end for skin penetration, a finishing part to close an accommodation space, etc. may be prepared as a metal region. Moreover, in order to exactly define a leakage point of an active ingredient such as a vaccine in the accommodation space, the metal region may be formed such that a first leakage point or an nth leakage point is provided in the metal region.

In addition, the biodegradable polymer material part adopted to allow the entire capsule, which still remains after the active ingredient has completely flowed out, to be degraded and absorbed in the biological tissues, may be prepared by applying bioabsorbable materials well known in the art, for example, polydioxanone (PDO), polylactic acid (PLA), poly-L-lactic acid (PLLA), polyglycolic acid (PGA), glycolide-lactide copolymer (PGLA), glycolide-caprolactone copolymer (PGLC), and the like. Unlike the aforementioned biodegradable metal, the polymer material has soft and flexible properties, and thus may achieve useful effects in relation to enlarged capacity of the active ingredient. This will be described below in more detail with reference to preferred embodiments of the present invention.

With regard to the above-described technique for controlling a degradation rate of biodegradable metal, the biodegradable metal is represented by the following Formula 1, and may be configured such that the biodegradable metal is inserted into subcutaneous biological tissues and then is absorbed and degraded to release magnesium or zinc metal ions and a degradation product into the body.

[Formula 1] MgaZnbXc

wherein each of a, b, and c is a weight percent of each component; a + b + c = 100 wt%; a or b is the largest value in the range of 0 ≤ a ≤ 100, 0 ≤ b ≤ 100, and 0 ≤ c ≤ 40; and X is one or more selected from the group consisting of Ca, Fe, Mn, Si, Na, Zr, Ce and P.

The biodegradable metal applied to the bioabsorbable drug delivery capsule preferably contains Mg or Zn in the largest content, accordingly, each of a, b and c in Formula 1 is a weight percent of each component, a + b + c = 100% by weight, i) 90 ≤ a ≤ 100, 0 ≤ b ≤ 10, 0 ≤ c ≤ 40 or ii) 0≤ a ≤ 10, 90 ≤ b ≤ 100, 0 ≤ c ≤ 40, and X is preferably at least one selected from the group consisting of Ca, Fe, Mn, Si, Na, Zr, Ce and P.

In other words, the bioabsorbable drug delivery capsule of the present invention may consist of at least two metals represented by the above formula to produce a galvanic circuit of metal components, which in turn may be regulated to control a degradation rate of the casing in the biological tissues according to the purpose of active ingredients (such as vaccine) to act in vivo.

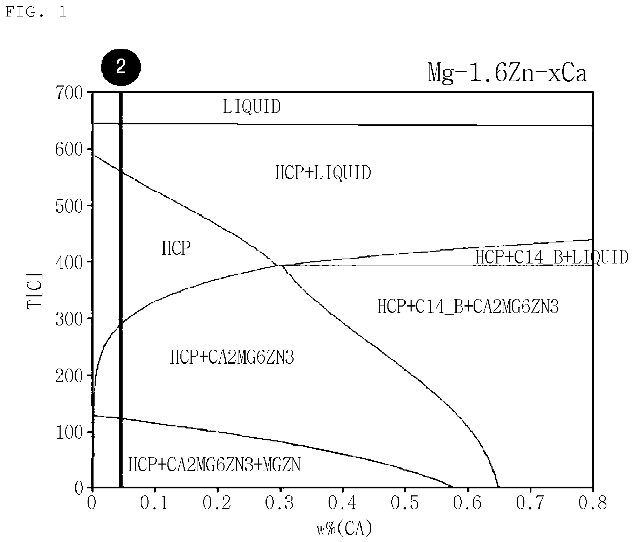

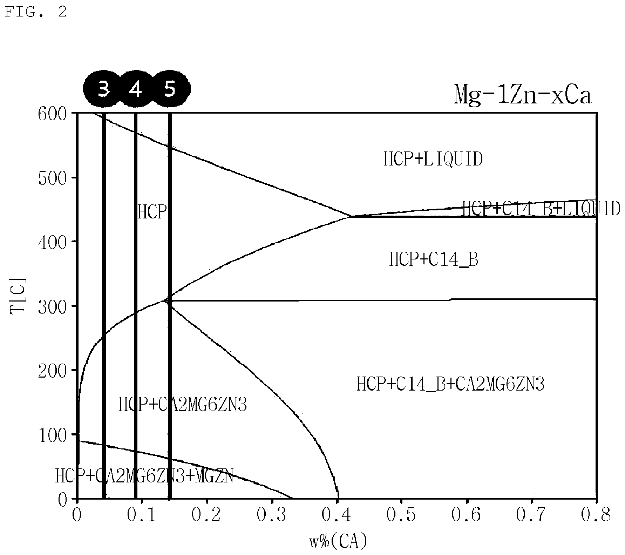

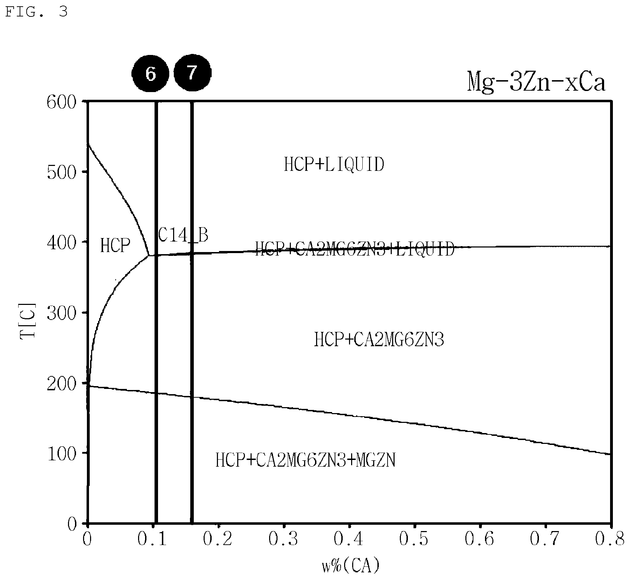

FIGS. 1 to 3 illustrates state diagrams in relation to contents of magnesium, zinc and calcium, wherein the numbers 2 to 7 indicated therein are the numbers of the samples used in the illustrated experimental examples. As shown in the figures, magnesium, zinc and calcium may be present in different states according to contents thereof. For instance intermetallic compounds such as Mg2Ca (C14_B), Ca2Mg6Zn3, MgZn, etc. may generate a galvanic circuit to increase the degradation rate as compared to a single material.

The biodegradable metal applied to the bioabsorbable drug delivery capsule according to the present invention may have a structure in which a second metal different from the biodegradable metal represented by Formula 1 is coated on the surface thereof to generate a galvanic circuit. In this case, the second metal may include, for example, sodium, magnesium, potassium, iron, nickel, zinc, gallium, selenium, strontium, zirconium, molybdenum, niobium, tantalum, titanium, silicon, silver, gold, manganese, calcium, and the like, but is not limited thereto. At this time, when the biodegradable metal is prepared using iron (Fe), a stainless steel component should not be contained.

Experimental basis for rapid increase in the degradation rate when an intermetallic compound phase such as Mg2Ca (C14_B), Ca2Mg6Zn3, MgZn, etc. is formed, may be supported by Experimental Example 1 below.

As described above, based on the results obtained by experiments under various conditions in regard to the degradation rates of various biodegradable metals under the same environment in vivo, the present invention may provide a subcutaneously implantable and bioabsorbable capsule for drug delivery, which can repeatedly deliver a desirable amount of an active ingredient in a casing into biological tissues at an appropriate time and at an appropriate time interval. Eventually, applying a chemical constitutional composition of the biodegradable metal and a thickness of the capsule may control a point in time of drug release.

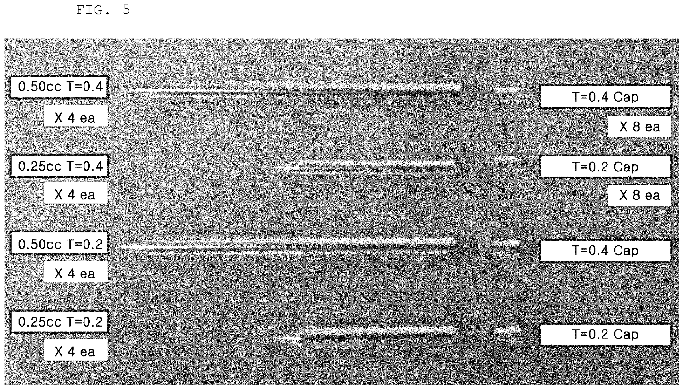

FIG. 5 is a sample photograph of the bioabsorbable drug delivery capsules according to the first embodiment of the present invention, wherein specific forms for the aforementioned functions are illustrated.

As shown, in the case of the bioabsorbable drug delivery capsule according to the first embodiment of the present invention, the entire body is made of a biodegradable metal and has an accommodation space inside the casing in order to receive an active ingredient such as a vaccine.

Further, the drug delivery capsule of the first embodiment has a structure advantageous for penetrating into the subcutaneous tissue since a front end having a tapered shape made of a biodegradable metal having sufficient strength to penetrate the skin is provided. At this time, in order to facilitate an insertion operation, a launch device for pushing the drug delivery capsule in the above form into the skin may be further provided.

In addition, the casing of the capsule according to the present invention may have a variety of sizes depending on a required amount of the active ingredient, or a plurality of casings may be inserted if necessary.

Furthermore, the casing may be configured such that, after filling the accommodation space, as shown in the drawing, with the active ingredient, the active ingredient in the accommodation space is encapsulated and maintained in the capsule using a plug to block the accommodation space until a predetermined region of the casing is degraded in the biological tissues to afford a communication portion.

Vaccination experiments were implemented on an experimental pig provided by the Livestock Science Institute with the samples prepared through the first embodiment, details of which will be described later through Experimental Example 2.

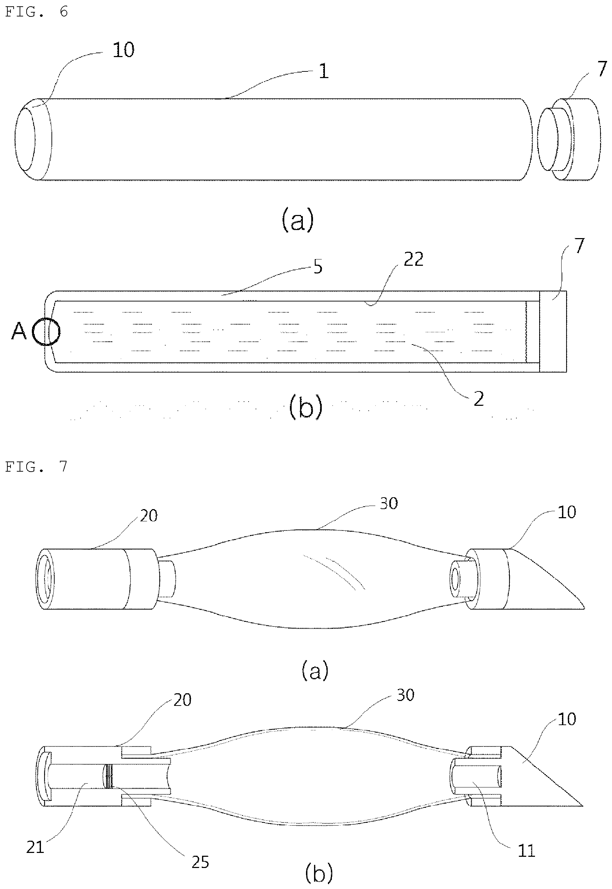

FIG. 6 is a modified embodiment of the first embodiment, illustrating the form of a drug delivery capsule according to the second embodiment.

As shown in the figure, the drug delivery capsule according to the second embodiment is almost similar to the first embodiment except for the shape of the front end 10. Further, as shown in FIG. 6(b),the drug delivery capsule has a structure in which the accommodation space 22 is blocked by the plug 7 after injecting the active ingredient 2 such as the drug.

According to one object of the present invention, in order to flow the active ingredient 2 into the biological tissues at an appropriate rate and at an appropriate time, the second embodiment was given to clearly illustrate a technical example to specifically define a leakage point by regulating a degradation region and a degradation rate of the casing 1. FIG. 6(b) is a cross-sectional view clearly illustrating an internal structure of the drug delivery capsule according to the second embodiment, specifically, showing a point A at which an area thinner than other areas of a partition wall 5 in the casing is formed.

That is, assuming that the entire area of the casing 1 is made of a biodegradable metal with the same constitutional composition, under the same conditions in vivo, the point A where the casing partition wall 5 has the smallest thickness may be firstly communicated during degradation of the casing 1, and leakage holes are formed in desired shape so that the active ingredient 2 may adjustably flow out at a desired rate.

Further, although the present embodiment has exemplified the capsule provided with only one accommodation space 22, this accommodation space 22 may be divided into two or more spaces so that the active ingredient 2 can leak into the living body sequentially at desired times by configuring a leakage point of each accommodation space in the aforementioned manner.

In other words, it is possible to set the order of leakage of the active component in such a way that the first leakage point is a corresponding point of the accommodation space 22 including the thinnest partition wall and the second leakage point is a corresponding point of the accommodation space 22 including the next thinnest partition wall. For instance, for a vaccine requiring three inoculations, if a first inoculation is carried out simultaneously with inserting the drug delivery capsule provided with two accommodation spaces 22 according to the present invention into the biological tissues, the active ingredient may leak to exert effects of a second inoculation at the first leakage point after a desired and predetermined period of time, and then, the active ingredient may leak again at the second leakage point after a predetermined period of time, so as to exert effects of a third inoculation and complete a process for antibody formation.

On the other hand, FIG. 6 explains an example of regulating a leakage amount and a leakage time of the active ingredient by adjusting a thickness of the casing partition wall 5. However, as will be apparent through the above Formula 1 and the experimental example described later, theoretical and experimental grounds in relation to adjustment of constitutional compositions of metals which in turn can control a degradation rate of biodegradable metals in vivo, have been ensured. Therefore, by varying the constitutional composition of the biodegradable metals in specific regions or portions of the casing 1, the first and nth leakage points may be set in the same manner as the adjustment of thickness and, at the same time, separate accommodation spaces 22 may be configured to allow the active ingredient 2 contained in each of the accommodation spaces to leak at a desired rate and at a desired time point.

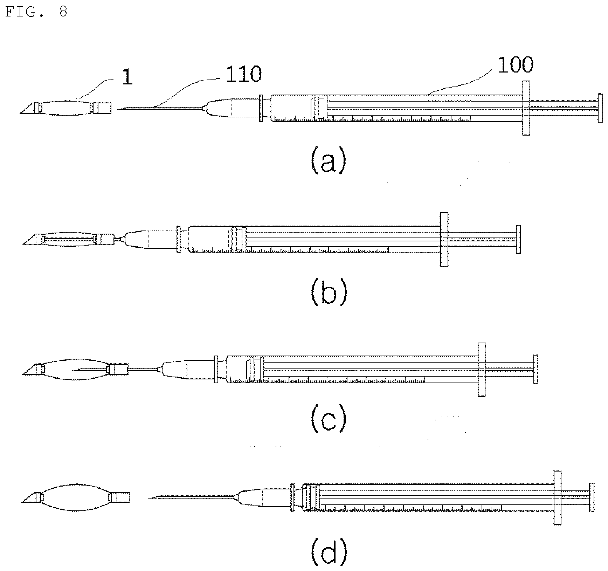

FIGS. 7 to 8 illustrate a third embodiment among the preferred embodiments of the present invention, specifically, FIG. 7 is a view of the bioabsorbable drug delivery capsule according to the third embodiment of the present invention, while FIG. 8 explains a process of applying the bioabsorbable drug delivery capsule according to the third embodiment of the present invention in vivo.

As briefly mentioned above and shown in detail in FIG. 7, the bioabsorbable drug delivery capsule according to the present invention may be provided in the form of a combination of a biodegradable metal and a biodegradable polymer material.

That is, as shown in FIG. 7, the front end 10 and the nodule 20 of the structure able to penetrate the skin in a tapered shape may be made of a relatively high strength biodegradable metal according to Formula 1 above, and an expansion 30 made of a soft biodegradable polymer material may be coupled between the front end 10 and the nodule 20.

In the first and second embodiments described above, the whole material consists of a solid metal material, and therefore, casings 1 in different sizes should be provided according to an injected amount of the active ingredient 2 in vivo. Further, with a large amount of the active ingredient 2, the casing 1 is enlarged or the number of casings 1 to be inserted is increased, making insertion difficult, in addition, possibly causing a disadvantage such as an impact to the living body.

Therefore, when the bioabsorbable drug delivery capsule according to the present invention has the same structure as in the third embodiment, the active ingredient 2 can be mostly accommodated in the soft expansion30, thereby ensuring a further advantage of decreasing an insertion size. Further, the advantage described above of desirably setting a leakage time and a leakage amount of the active ingredient by controlling a degradation rate of the metal region can be applied as is.

In other words, the expansion 30 may be prepared by applying an elastic-expandable polymer material in a tube or bag shape. However, even if not highly elastic, the expansion 30 can be inserted into biological tissues in a crumpled or compressed state as long as it is made of a soft material. Further, polydioxanone (PDO), polylactic acid (PLA), poly-L-lactic acid (PLLA), polyglycolic acid (PGA), glycolide-lactide copolymer (PGLA) and glycolide-caprolactone copolymer (PGCL) and the like, which are widely known as components possibly degraded and eventually absorbed in vivo, may be applied.

As shown in FIG. 7, the drug delivery capsule according to the third embodiment of the present invention includes configurations for: preventing the active ingredient in the expansion 30 from being discharged to the outside; injecting the active ingredient 2 into the casing 1 of the drug delivery capsule; preventing the injected active ingredient 2 from escaping; penetrating the skin; cooperating with a separate insertion means when inserting into the skin; and coupling the metal region with the expansion 30, and the like.

First, with regard to the configuration for injecting the active ingredient 2 into the casing 1 of the drug delivery capsule, as shown in FIG. 7(b),which is a cross-sectional view of the third embodiment, an inlet 21 through which a drug and the like can be injected is provided. Drug injection through the inlet 21 may be performed in different manners. Alternatively, as described later with reference to FIG. 8 or the like, the existing syringe 100 or the like can also be used.

On the other hand, the function of preventing the injected active ingredient 2 from escaping is achieved by a backflow preventing means 25 disposed in the nodule 20.The backflow preventing means 25 may be provided with a well-known backflow preventing valve structure and, in the simplest form, a backflow preventing function by an elastic membrane to close the inlet through elastic force may be applied.

The skin penetrating function is achieved by the front end 10 provided with a tip member.

A method of inserting the capsule according to the present invention into the biological tissues by penetrating the skin using morphological features of the front end 10 may be achieved by a separate auxiliary tool and, by way of example, FIG. 8 illustrates a process of inserting the capsule according to the third embodiment of the present invention into the skin, wherein the above-described inlet 21 and the backflow preventing means 25 are configured in the form for accommodating the injection needle 110 of a syringe 100. In particular, an injection needle seat 11 on which an end of the injection needle 110 is also seated, may be provided inside the front end 10 so as to stably secure a skin insertion direction of the front end 10 when mounting the syringe 100.

As shown in FIG. 7(b), the configuration for coupling the metal region and the expansion 30 may also be obtained through forced-fit coupling of a fixing ring, which can be made of a biodegradable metal material secured to both ends of the tubular expansion 30 while placing both ends on the outside of the front end 10 or one end of the nodule 20.

FIG. 8 is a view illustrating a process of applying the drug delivery capsule in vivo according to the embodiment of FIG. 7. As shown in FIG. 7, the casing 1 has a shape corresponding to the injection needle 110 of the typical syringe 100.

Accordingly, as shown in FIG. 8(a), the casing 1 is coupled to the injection needle 110 in a state in which an appropriate amount of the active ingredient 2 to be injected has first entered the syringe 100.

Subsequently, in the state in which the casing 1 and the syringe 100 are coupled as shown in FIG. 8(b), an insertion process is performed through the skin using the front end 10 of the casing 1.

After the casing 1 is inserted at a desired site of the biological tissues inside the skin, the active ingredient 2 starts to be injected into the accommodation space of the casing 1 by pressing a piston of the syringe 100 as shown in FIG. 8(c).

When injection of the active ingredient 2 is completed, the expansion 30 of the drug delivery capsule according to the third embodiment is in a maximally swollen state. On the other hand, when the syringe 100 is removed as shown in FIG. 8(d), the active ingredient 2 is kept contained in the casing 1 by the backflow preventing means 25 described in FIG. 7.

Thereafter, the active ingredient 2 at the desired first leakage point begins leaking after lapse of a predetermined time, thereby achieving the same effects as a second inoculation of vaccine.

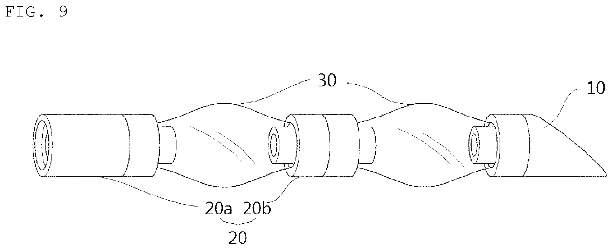

FIG. 9 is a view of the bioabsorbable drug delivery capsule according to a fourth embodiment as a modification of the third embodiment shown in FIGS. 7 and 8.

The fourth embodiment shown in FIG. 9 has two expansions 30 and, for this purpose, the nodule 20 also includes a distal nodule 20a and an intermediate nodule 20b.

Such a configuration is inserted during first inoculation and is meaningful as an embodiment for simultaneously realizing second and third inoculation effects at a predetermined interval without additional inoculation.

### [Detailed Description of Preferred Embodiments of Invention]

Hereinafter, in order to more clearly clarify the effects of the configurations described above and actual configurations in the various embodiments, experimental examples implemented by the study of the present invention will be described in detail.

### [Experimental Examples]

Hereinafter, the present invention will be described in more detail through experimental examples. These experimental examples are only for illustrating the present invention, and it will be apparent to those skilled in the art that the scope of the present invention should not be interpreted as being limited by these experimental examples.

### (*) Experimental Example 1: Evaluation of Metal Sheet Degradation Rate

### Samples 1-9: Metal sheets made of magnesium or zinc as main materials

With the constitutional compositions (% by weight: wt.%) of Table 1, metal sheets were prepared (with a dimension of left and right widths of 60 mm, 1.5 mm; obtained by extrusion at a rate of 0.1 mm/s after casting in a high frequency melting furnace).

**[TABLE 1]**

| Sample | Mg | Ca | Zn |
|---|---|---|---|
| 1 | 99.99* | - | - |
| 2 | 98.35 | 0.05 | 1.60 |
| 3 | 98.95 | 0.05 | 1.00 |
| 4 | 98.9 | 0.10 | 1.00 |
| 5 | 98.85 | 0.15 | 1.00 |
| 6 | 96.9 | 0.10 | 3.00 |
| 7 | 96.85 | 0.15 | 3.00 |
| 8 | 0.00 | 0.00 | 99.99* |
| 9 | 10.00 | 0.00 | 90.00 |

| | | | |
|---|---|---|---|
| (*) Pure metals containing inevitable impurities generated during manufacturing | | | |

After immersing each of the metal sheets prepared in Samples 2 to 7 in an eudiometer containing a bio-like fluid having the constitutional composition of Table 2, a degradation rate was evaluated on the basis of an amount of hydrogen generated relative to immersion time, and the evaluated results are shown in FIG. 5.

**[TABLE 2]**

| Component | Molar concentration (mM/L) | Mass (g) |
|---|---|---|
| CaCl₂ . 2H₂O | 1.26 | 0.185 |
| KCl | 5.37 | 0.400 |
| KH₂PO₄ | 0.44 | 0.060 |
| MgSO₄.H₂O | 0.81 | 0.200 |
| NaCl | 136.89 | 8.000 |
| Na₂HPO₄ . 2H₂O | 0.34 | 0.060 |
| NaHCO₃ | 4.17 | 0.350 |
| D-glucose | 5.55 | 1.000 |

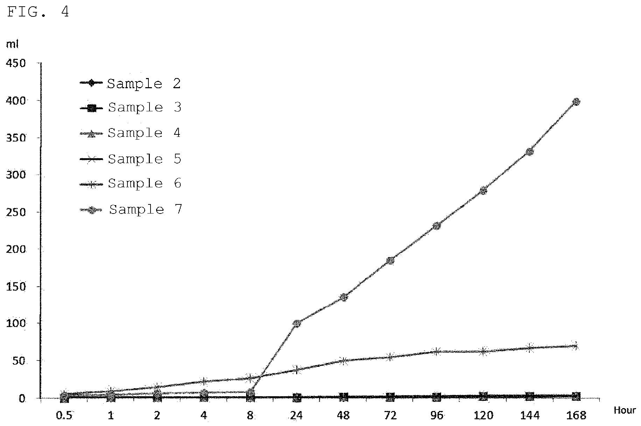

As shown in FIG. 4, the metal sheets of Samples 2 to 5 in which the Mg2Ca phase is not generated had an amount of hydrogen gas generated by stable degradation. However, a hydrogen gas generation amount of the metal sheets in Samples 6 and 7 in which the Mg2Ca phase is generated was significantly higher than other examples, thereby demonstrating increase in degradation rate. From the result, it could be seen that the degradation rate of the biodegradable metal can be controlled by adjusting the constitutional composition in Formula 1.

### (*) Experimental Example 2: Experiment for Formation of Pig Foot-and-Mouth Disease Antibody

### [Primary antibody formation experiment]

At the end of 2016, experiments were conducted for 9 weeks using sixteen (16) pigs all 8 weeks of age. The animals were divided into a conventional injection group as the control and an experimental group with insertion of the drug delivery capsule according to the first embodiment of the present invention, and an extent of antibody formation was measured and compared between both of the above groups.

ELISA was used as an immunoassay method to identify formation of vaccine antibody (SP). When the assayed value is 50 or more, the SP is determined as a positive antibody.

The drug delivery capsule includes pure magnesium, has an accommodation space of 3.40 Pi(π) and a size of 54.70 mm, each carrying 0.5 cc of the same type of vaccine.

Test axis: 16 pigs of 8 weeks age, experiment for 9 weeks
Group 1 (5 pigs): application of foot-and-mouth vaccination (muscle) (8 and 12 weeks after birth)
Group 2 (5 pigs): application of foot-and-mouth vaccination (subcutaneous) (8 and 12 weeks after birth)
Group 3 (3 pigs): foot-and-mouth vaccination (subcutaneous) + 4-week capsule vaccination (8 weeks after birth)
Group 4 (3 pigs): foot-and-mouth vaccination (subcutaneous) + 2-week capsule vaccination (8 weeks after birth)

**[TABLE 3]**

| Assay group | 8 (weeks age) | 9 | 10 | 11 | 12 | 13 | 14 | 15 | 16 | 17 |
|---|---|---|---|---|---|---|---|---|---|---|
| | 0 (time) | 1 | 2 | 3 | 4 | 5 | 6 | 7 | 8 | 9 |
| Group 1 | 58 | 48 | 58 | 59 | 59 | 87 | 82 | 80 | 75 | 78 |
| Group 2 | 47 | 41 | 54 | 55 | 54 | 81 | 79 | 80 | 77 | 75 |
| Group 3 | 57 | 52 | 47 | 40 | 39 | 63 | 65 | 67 | 63 | 67 |
| Group 4 | 57 | 55 | 52 | 49 | 50 | 77 | 80 | 85 | 83 | 85 |

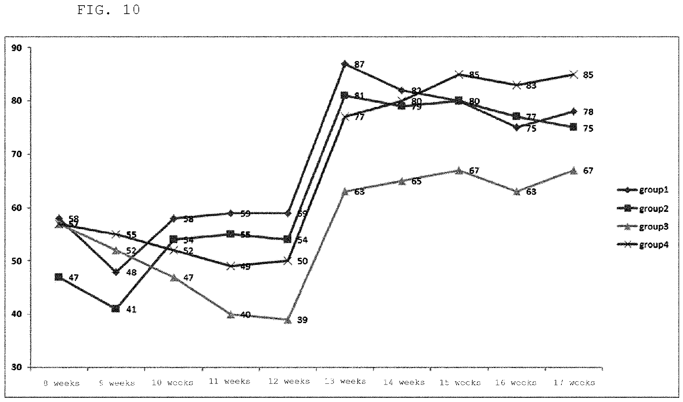

A graphical representation of the numerical values summarized in the above table is shown in FIG. 10. As could be seen from the table and the graph of FIG. 10, sufficient antibody formation was observed in both group 3 and group 4 to which the capsule according to the present invention was applied. Specifically, group 4 with application of the 2-week digestion capsule exhibited higher antibody formation effects than the conventional injection method. In the case of the injection method, an immune-quantitative value was slightly lowered after the second inoculation. On the contrary, a result of minutely continued increase in the immune-quantitative value was observed in group 3 and group 4. This is presumed to be because the required vaccine is slightly leaking rather than leakage at once or, otherwise, because of diverse causes including synergistic effects due to by-products from the degradation of biodegradable metals.

### [Secondary antibody formation experiment]

At the end of 2017, experiments were carried out using fourteen (14)pigs all 8weeks of age as shown in Table 4 below. The animals were divided into a conventional injection group as the control and an experimental group with insertion of the drug delivery capsule according to the first embodiment of the present invention, and an extent of antibody formation was measured and compared between both of the above groups.

ELISA was used as an immunoassay method to identify formation of vaccine antibody (SP).

The drug delivery capsule includes pure magnesium, has an accommodation space of 3.40 Pi(π) and a size of 54.70 mm, each carrying 0.5 cc of the same type of vaccine.

**[TABLE 4]**

| Experiment: antibody formation rate assay | Control | Group 1 |
|---|---|---|
| | 2 pigs | 4 pigs |
| 7 weeks after birth | Pig transport and stocking | |
| 8 weeks after birth (0 week) | Vaccine, blood collection | Vaccine + capsule vaccine transplant, blood collection |
| 9 weeks after birth (1 week) | Blood collection | Blood collection |
| 10 weeks after birth (2 weeks) | Blood collection | Blood collection |
| 11 weeks after birth (3 weeks) | Blood collection | Blood collection |
| 12 weeks after birth (4 weeks) | Vaccine, Blood collection | Blood collection |
| 13 weeks after birth (5 weeks) | Blood collection | Blood collection |
| 14 weeks after birth (6 weeks) | Blood collection | Blood collection |
| 15 weeks after birth (7 weeks) | Blood collection | Blood collection |
| 16 weeks after birth (8 weeks) | Blood collection | Blood collection |
| 17 weeks after birth (9 weeks) | Blood collection | Blood collection |
| 18 weeks after birth (10 week) | Blood collection | Blood collection |
| 28 weeks after | Blood collection, | Blood collection, |
| birth | whole butchery (sacrifice) | whole butchery (sacrifice) |

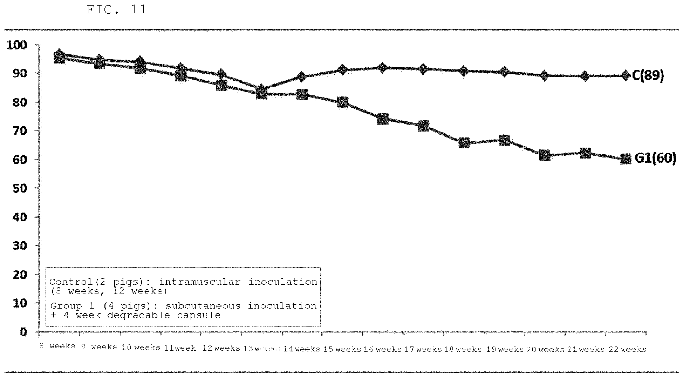

A graphical representation of the numerical values summarized in the above table is shown through FIG. 11.

As could be seen from the graph of FIG. 11, sufficient antibody formation was observed in group 1 to which the capsule according to the present invention was applied, and this result was substantially the same as in the control group which also received the muscle inoculation method (8 weeks and 12 weeks). Specifically, group 4 with application of the 2-week digestion capsule exhibited higher antibody formation effects than the conventional injection method.

As such, specific parts of the present invention have been described in detail through the preferred examples, and the above specific description would be merely given for preferred embodiments to those skilled in the art. Therefore, it will be obviously understood that the scope of the present invention is not particularly limited by the above preferred embodiments. Accordingly, the substantial scope of the present invention will be defined by the appended claims and their equivalents.

## Claims

1. A subcutaneously implantable and bioabsorbable capsule for drug delivery ("bioabsorbable drug delivery capsule"), which is inserted into subcutaneous biological tissues to deliver an active ingredient, comprising: an accommodation space for the active ingredient; and a casing for blocking the accommodation space from an outside in order to prevent the active ingredient from leaking to the outside,
wherein the casing is composed of a bioabsorbable material to be degraded inside the biological tissues, so as to allow the active ingredient contained in the accommodation space to be discharged to the outside and then flow into the biological tissues.

2. The bioabsorbable drug delivery capsule according to claim 1, wherein the bioabsorbable material of the casing includes a biodegradable metal represented by the following Formula 1:
[Formula 1] MgaZnbXc
wherein each of a, b, and c is a weight percent of each component; a + b + c = 100 wt%; a or b is the largest value in the range of 0 ≤ a ≤ 100, 0 ≤ b ≤ 100, and 0 ≤ c ≤ 40; and X is one or more selected from the group consisting of Ca, Fe, Mn, Si, Na, Zr, Ce and P.

3. The bioabsorbable drug delivery capsule according to claim 2, wherein, in Formula 1, each of a, b and c in Formula 1 is a weight percent of each component, a + b + c = 100% by weight, i) 90 ≤ a ≤ 100, 0 ≤ b ≤ 10, 0 ≤ c ≤ 40, or ii) 0≤ a ≤ 10, 90 ≤ b ≤ 100, 0 ≤ c ≤ 40, and X is at least one selected from the group consisting of Ca, Fe, Mn, Si, Na, Zr, Ce and P.

4. The bioabsorbable drug delivery capsule according to claim 2, wherein the biodegradable metal is pure Mg containing inevitable impurities.

5. The bioabsorbable drug delivery capsule according to claim 2, wherein the biodegradable metal is pure Zn containing inevitable impurities.

6. The bioabsorbable drug delivery capsule according to any one of claims 2 and 5, wherein at least a portion of the casing is a metal region made of the biodegradable metal; a first leakage point, in which the active ingredient in the accommodation space leaks first to the outside, is formed during degradation of the bioabsorbable material in the metal region; and an n^{th} leakage point (n being an integer of 2 or more), in which leakage of the active ingredient sequentially occurs after the leakage at the first leakage point, can be formed at another site of the casing including the metal region.

7. The bioabsorbable drug delivery capsule according to claim 6, wherein the first leakage point or the n^{th} leakage point is formed by appropriately arranging a constitutional composition of the biodegradable metal in the metal region, wherein the above constitutional composition allows two or more metal phases to generate a galvanic circuit according to Formula 1, thereby accelerating a degradation rate.

8. The bioabsorbable drug delivery capsule according to claim 6, wherein the first leakage point or the n^{th} leakage point is **characterized in that** a thickness of a casing wall between the accommodation space of the casing and the outside is adjusted to define the thinnest portion (that is, the casing wall portion having the smallest thickness) as the first leakage point, followed by sequentially forming the n^{th} leakage point according to the thickness of the casing wall.

9. The bioabsorbable drug delivery capsule according to claim 6, wherein the accommodation space including the first leakage point or the n^{th} leakage point includes isolated spaces, which are formed in the casing in correspondence with the first leakage point or the n^{th} leakage point, respectively, wherein the active ingredient does not communicate with the isolated spaces.

10. The bioabsorbable drug delivery capsule according to any one of claims 2 to 5, wherein the bioabsorbable drug delivery capsule of the present invention consists of: a front end made of the biodegradable metal; an expandable part ("expansion") made of a soft biodegradable polymer material; and at least one nodule made of the biodegradable metal, which is connected to at least one end of the expansion
wherein the expansion is formed in a tube or bag shape that can accommodate the active ingredient therein, and has a configuration such that the front end or the nodule communicates with both ends of the expansion.

11. The bioabsorbable drug delivery capsule according to claim 10, wherein the front end is provided with a tip member to penetrate the skin while being inserted subcutaneously, and the nodule has an inlet through which the active ingredient can be injected, as well as a backflow preventing member to prevent the injected active ingredient from being discharged to the outside.

12. The bioabsorbable drug delivery capsule according to claim 11, wherein the inlet of the nodule has a tubular shape through which an injection needle can penetrate so that the inlet of the nodule can be used by mounting the injection needle of a drug injection means such as a syringe thereon, the backflow preventing means has a backflow preventing valve structure such as an elastic membrane so that the active ingredient injected from the drug injection means after removing the injection needle can be prevented from being discharged to the outside, and
an injection needle seat is further provided inside a connection part between the front end and the expansion, wherein the injection needle seat stably secures a skin insertion direction of the front end when mounted on the injection needle.

13. The bioabsorbable drug delivery capsule according to claim 10, wherein the expansion is made of at least one biodegradable polymer material selected from the group consisting of polydioxanone (PDO), polylactic acid (PLA), poly-L-lactic acid (PLLA), polyglycolic acid (PGA), glycolide-lactide copolymer (PGLA) and glycolide-caprolactone copolymer (PGCL).
